# FASCICULE DE BREVET EUROPEEN

(11) **EP 2 488 633 B1**
(45) Date de publication et mention de la délivrance du brevet: **09.10.2013**
(21) Numéro de dépôt: 10774280.1
(22) Date de dépôt: 15.10.2010
(51) Int. Cl.: C12N 7/02

(54) **METHODE IN VITRO D'OBTENTION DE FIBROBLASTES INTRAHEPATIQUES INFECTES PAR LE VIRUS DE L'HEPATITE C**
IN VITRO VERVAHREN ZUR GEWINNUNG VON MIT DEM HEPATITISVIRUS C INFIZIERTEN INTRAHEPATISCHEN FIBROBLASTEN
IN VITRO METHOD FOR OBTAINING INTRAHEPATIC FIBROBLASTS INFECTED WITH HEPATITIS C VIRUS

(30) Priorité: 16.10.2009 FR 0904962
(43) Date de publication de la demande: 22.08.2012
(73) Titulaire: Université Paris Descartes, 75006 Paris (FR); Assistance Publique Hôpitaux De Paris, 75004 Paris (FR)
(72) Inventeur: CALMUS, Yvon, F-75014 Paris (FR); CONTI, Filomena, F-78140 Velizy (FR)
(74) Mandataire: Métay, Émeline
(86) Numéro de dépôt international: PCT/IB2010/054687
(87) Numéro de publication internationale: WO 2011/045772

(56) Documents cités:
- STEFFAN A.M. ET AL.: "Ultrastructural observations in hepatitis C virus-infected lymphoid cells.", MICROB. INFECT., vol. 3, 2001, pages 193-202, XP002581716,
- BARTOLOMÉ J. ET AL.: "Detection of Hepatitis C Virus in thyroid tissue from patients with chronic HCV infection.", J. MED. VIROL., vol. 80, 2008, pages 1588-1594, XP002581717,
- AOUDJEHANE L. ET AL.: "Interleukin-4 induces the activation and collagen production of cultured human intrahepatic fibroblasts via the STAT-6 pathway.", LAB. INVEST., vol. 88, 2008, pages 973-985, XP002581718,
- SHACKEL N A ET AL: "INSIGHTS INTO THE PATHOBIOLOGY OF HEPATITIS C VIRUS-ASSOCIATED CIRRHOSIS ANALYSIS OF INTRAHEPATIC DIFFERENTIAL GENE EXPRESSION", AMERICAN JOURNAL OF PATHOLOGY, AMERICAN SOCIETY FOR INVESTIGATIVE PATHOLOGY, US, vol. 160, no. 2, 1 février 2002 (2002-02-01), pages 641-654, XP001176854, ISSN: 0002-9440

### SEQUENCE LISTING

<110> UNIVERSITE PARIS DESCARTES
<120> METHODE DE PRODUCTION DE FIBROBLASTES INTRAHEPATIQUES INFECTES PAR LE VIRUS DE L'HEPATITE C
<130> F1405-6WO
<160> 14
<170> PatentIn version 3.3
<210> 1
   <211> 21
   <212> DNA
   <213> Artificial
<220>
   <223> Amorce sens GAPDH
<400> 1
   acagtccatg ccatcactgc c 21
<210> 2
   <211> 21
   <212> DNA
   <213> Artificial
<220>
   <223> Amorce antisens GAPDH
<400> 2
   gcctgcttca ccaccttctt g 21
<210> 3
   <211> 19
   <212> DNA
   <213> Artificial
<220>
   <223> Amorce sens collagène de Type I
<400> 3
   cctcaagggc tccaacgag 19
<210> 4
   <211> 20
   <212> DNA
   <213> Artificial
<220>
   <223> Amorce antisens collagène de Type **I**
<400> 4
   tcaatcactg tcttgcccca 20
<210> 5
   <211> 20
   <212> DNA
   <213> Artificial
<220>
   <223> Amorce sens collagène de Type IV
<400> 5
   atgtcaatgg cacccatcac 20
<210> 6
   <211> 20
   <212> DNA
   <213> Artificial
<220>
   <223> Amorce antisens collagène de Type IV
<400> 6
   cttcaaggtg gacggcgtag 20
<210> 7
   <211> 18
   <212> DNA
   <213> Artificial
<220>
   <223> Amorce sens Alpha-SMA
<400> 7
   tgaagagcat cccaccct 18
<210> 8
   <211> 18
   <212> DNA
   <213> Artificial
<220>
   <223> Amorce antisens Alpha-SMA
<400> 8
   acgaaggaat agccacgc 18
<210> 9
   <211> 22
   <212> DNA
   <213> Artificial
<220>
   <223> Amorce sens HNF-1 beta
<400> 9
   gaaacaatga gatcacttcc tc 22
<210> 10
   <211> 22
   <212> DNA
   <213> Artificial
<220>
   <223> Amorce antisens HNF-1 beta
<400> 10
   ctttgtgcaa ttgccatgac tc 22
<210> 11
   <211> 20
   <212> DNA
   <213> Artificial
<220>
   <223> Amorce sens CP450
<400> 11
   agcacaactc tgagatatgg 20
<210> 12
   <211> 20
   <212> DNA
   <213> Artificial
<220>
   <223> Amorce antisens CP450
<400> 12
   atagtcactg tacttgaact 20
<210> 13
   <211> 29
   <212> DNA
   <213> Artificial
<220>
   <223> Amorce sens Albumine
<400> 13
   ttaggatccc ccaggaagac atcctttgc 29
<210> 14
   <211> 18
   <212> DNA
   <213> Artificial
<220>
   <223> Amorce antisens Albumine
<400> 14
   cctgagccag agatttcc 18

## Description

Le virus de l'hépatite C est un virus enveloppé avec un génome ARN simple brin de polarité positive. Il appartient au genre des hepacivirus, dont il est le seul membre, et fait partie de la famille des *Flaviviridae.* La réplication de l'ARN génomique passe par l'intermédiaire d'un brin anti-génomique de polarité négative, qui servira à son tour de matrice pour la synthèse d'ARNs génomiques. Les ARNs génomiques néosynthétisés serviront, soit à la synthèse d'une polyprotéine clivée de façon co- et post-traductionnnelle par des protéases cellulaires et virales afin de produire les protéines virales structurales et non-structurales, soit à la synthèse de nouveaux brins d'ARN de polarité négative, ou bien seront encapsidés suite à l'oligomérisation de la protéine de capside. Les nucléocapsides formées dans le cytoplasme acquièrent leur enveloppe par bourgeonnement au niveau du réticulum endoplasmique où sont retenues les glycoprotéines E1 et E2 puis les particules virales sont libérées dans le milieu extérieur.

L'infection par ce virus représente un important problème de santé publique au vu de la forte prévalence de l'infection et du risque important d'évolution vers une hépatite chronique, estimé entre 50 et 80%. En effet, le virus de l'hépatite C (VHC) infecte environ 3 % de la population et est responsable d'approximativement 170 millions d'infections chroniques. L'infection chronique aboutit progressivement à une hépatite fibreuse susceptible d'évoluer en cirrhose et en hépatocarcinome.

Actuellement, le traitement antiviral le plus efficace consiste en une bithérapie reposant sur l'utilisation conjointe d'interféron alpha pégylé et d'un analogue nucléosidique, la ribavirine. Cependant, ce traitement n'est efficace que chez environ 50% des patients traités, et est par ailleurs mal toléré par de nombreux patients.

La lutte contre le virus de l'hépatite C reste donc un enjeu majeur de santé publique, et il est impératif de développer de nouvelles molécules ciblant le cycle viral, ou capables d'interrompre, voire d'empêcher, la survenue de fibroses induites lors de l'infection par le VHC.

La fibrose hépatique est la complication commune des maladies chroniques du foie, et en particulier de l'hépatite virale C. Elle est caractérisée par l'accumulation de matrice extracellulaire (MEC) composée principalement de collagènes de type I, III, IV et V. Au cours de la fibrogénèse hépatique, les cellules hépatiques étoilées (ou « HSC » pour « hepatic stellate cell ») et les fibroblastes intrahépatiques (FIH) sont activés ou se transdifférencient en myofibroblastes hépatiques qui acquièrent la capacité d'exprimer l'alpha-SMA (« smooth muscle alpha-actin »), lesquels produisent de la matrice extracellulaire (MEC) et inhibent la dégradation de cette dernière en dégradant les métalloprotéases.

Le mécanisme des lésions hépatiques induites lors de l'infection par le virus de l'hépatite C (VHC) est mal connu et le lien entre infection des hépatocytes par ce virus et activation de la fibrogénèse reste hypothétique. Il a notamment été proposé que l'inflammation chronique résultant de l'infection des hépatocytes par le VHC produise un stimulus qui induirait l'activation des fibroblastes intrahépatiques (FIH). Selon cette hypothèse, les cytokines libérées par les hépatocytes infectés par le VHC, les cellules endothéliales, les cellules de Kupffer ou les lymphocytes infiltrés pourraient être responsables de l'activation (Friedman SL., J. Biol. Chem., 275 : 2247-2250, 2000 ; Schulze-Krebs et al., Gastroenterology, 129(1) : 246-258, 2005).

Afin de développer des antiviraux ciblant efficacement la fibrose induite lors de l'infection par le VHC, il est nécessaire d'élucider les mécanismes à l'origine de cette fibrose viro-induite.

Les Inventeurs ont maintenant découvert que le VHC est capable d'infecter *in vivo* les fibroblastes intrahépatiques (FIH) et de les activer, ce qui suggère fortement que les hépatocytes pourraient jouer un rôle de réservoir du virus, mais que la fibrose serait due essentiellement à l'infection des fibroblastes. Cette découverte est surprenante puisque jusqu'à présent le VHC était connu pour infecter uniquement les hépatocytes, les monocytes, les lymphocytes et certaines cellules sécrétoires (Shimizu et al., Hepatology, 23 : 205-209, 1996 ; Wong et al., J. Virol., 75 : 1229-1235, 2001 ; Caussin-Schwemling et al., J. med. Virol.,65 : 14-22, 2001 ; Arrieta et al., Am. J. Pathol., 158 : 259-264, 2001 Steffan et al., Microb. Infect. 3 : 193-202, 2001 ; Bartolomé et al., J. Med. Virol. 80 : 1588-1594, 2008). Aucune donnée ne suggérait que les fibroblastes intrahépatiques sont sensibles et permissibles à l'infection par le VHC.

Ainsi, un premier objet de la présente invention concerne une méthode d'obtention de fibroblastes intrahépatiques infectés par le virus de l'hépatite C comprenant une étape d'isolement des fibroblastes intrahépatiques à partir d'un tissu hépatique provenant d'un patient VHC-positif.

On entend par « patient VHC-positif » toute personne qui, au jour du prélèvement de tissu hépatique, est infectée par le VHC. Il peut s'agir notamment d'un patient atteint d'une hépatite C chronique.

Les fibroblastes intrahépatiques peuvent être isolés par différentes méthodes connues en elles-mêmes, par exemple celle décrite par Tiggelman MBC et al. (J. Hepatol., 23 : 307-317, 1995) ou celle décrite par Win KM et al. (Hepatology, 18 : 137-145, 1993). Ces cellules peuvent aisément être caractérisées, et différenciées d'autres types cellulaires, notamment par la méthode décrite par Aoudjehane et al. (Lab. Invest., 88 : 973-985, 2008). D'autre part, les fibroblastes intrahépatiques étant des cellules activées exprimant l'alpha-SMA et la vimentine, ces marqueurs permettent de les caractériser, en association avec la présence de la molécule de surface CD90, et l'absence de la molécule CD31.

Les Inventeurs ont également constaté qu'il était possible d'infecter *in vitro* des fibroblastes intrahépatiques par le VHC. L'invention a donc également pour objet une autre méthode d'obtention de fibroblastes intrahépatiques infectés par le virus de l'hépatite C comprenant une étape de mise en contact *in vitro* de fibroblastes intrahépatiques avec des particules infectieuses du virus de l'hépatite C. Les stocks de particules virales infectieuses peuvent provenir de sérums de patients ou de culture *in vitro* du virus. Dans ce dernier cas, les particules infectieuses peuvent notamment être les particules VHC-JFH1 produites en transfectant l'ARN génomique transcrit à partir du plasmide pJFH-1 dans des cellules Huh-7.5 (Wakita et al., Nat. Med., 11 : 791-796, 2005). D'autres particules infectieuses que VHC-JFH1, exprimant des génotypes différents ou des génotypes chimériques, peuvent également être utilisées, notamment celles décrites par Gottwein et al. (Gastroenterology, 133 : 1614-1626, 2007) et Pietschmann et al. (P.N.A.S., 103 : 7408-7413, 2006).

Les fibroblastes intrahépatiques infectés par le virus de l'hépatite C, notamment ceux obtenus par les méthodes décrites ci-dessus, font également partie de la présente invention. On entend par « fibroblastes intrahépatiques infectés par le virus de l'hépatite C », non seulement les fibroblastes intrahépatiques infectés suite à la fixation du virus à la cellule et la pénétration de la particule virale dans la cellule, mais également toute cellule comprenant l'ARN génomique (brin de polarité positive) et/ou l'ARN anti-sens (brin de polarité négative) du VHC, ou tout autre ARN comprenant tout ou partie de l'ARN génomique du VHC et conservant la capacité de se répliquer dans la cellule, par exemple des réplicons bicistroniques décrits par Lohmann et al. (Science, 285 : 110-113, 1999).

Un autre objet de la présente invention concerne un procédé de réplication *in vitro* du génome du virus de l'hépatite C qui comprend une étape de culture *in vitro* de fibroblastes intrahépatiques isolés infectés par le virus de l'hépatite C. Avantageusement, les fibroblastes intrahépatiques isolés infectés sont obtenus via les méthodes selon l'invention définies précédemment. Les conditions optimales de culture peuvent aisément être déterminées par l'homme du métier par des travaux de routine.

En outre, les fibroblastes intrahépatiques étant infectés *in vivo* et étant responsables de la fibrose hépatique, il est essentiel de tester les fibroblastes intrahépatiques infectés comme cibles de médicaments anti-VHC ou de médicaments visant à réduire les lésions hépatiques induites par le VHC, et en particulier la fibrose.

L'invention concerne donc également une méthode de criblage de molécules anti-VHC comprenant les étapes suivantes :
- mise en présence de fibroblastes intrahépatiques infectés par un virus de l'hépatite C, tels que définis ci-dessus, avec la molécule à tester ;
- mesure de la réplication du génome du VHC et/ou de la production de particules du VHC, notamment dans le surnageant de culture.

Lorsque l'on mesure la réplication du génome viral, on quantifie le brin d'ARN de polarité positive et/ou le brin d'ARN de polarité négative du VHC.

La production de particules virales peut être mesurée par diverses méthodes connues en elles-mêmes, par exemple des techniques d'immunodétection, notamment par quantification des protéines structurales (C, E1, E2) dans le surnageant, ou par observation en microscopie électronique après enrichissement par gradient de densité (Lindenbach BD et al., Science, 309 : 623-626, 2005.

L'invention a également pour objet une méthode de criblage de molécules anti-fibrogénèse comprenant les étapes suivantes :
- mise en présence de fibroblastes intrahépatiques infectés par un virus de l'hépatite C tels que définis ci-dessus, avec la molécule à tester ;
- mesure de l'expression par lesdits fibroblastes intrahépatiques d'au moins un gène impliqué dans la fibrose.

On entend par « gène impliqué dans la fibrose » tout gène qui est exprimé dans les fibroblastes intrahépatiques activés et qui concourt à la fibrogénèse responsable de la fibrose hépatique (pour revue voir la publication World. J. Gastroenterol., 15(20) : 2433-2440, 28 mai 2009, et notamment Tableau 1). On citera notamment les gènes des collagènes de type I, III, IV et V.

Avantageusement, les deux méthodes de criblage ci-dessus comprennent en outre une étape de comparaison du résultat de la mesure obtenu avec les fibroblastes intrahépatiques infectés par le VHC mis en présence avec la molécule à tester avec celui obtenu avec des fibroblastes intrahépatiques infectés par le VHC n'ayant pas été mis en contact avec la molécule à tester.

Les principales classes de médicaments susceptibles d'être testés sont notamment i) les antiviraux, capables de réduire l'infection des hépatocytes, mais dont l'effet sur les fibroblastes est inconnu, ii) les médicaments antifibrosants, qui n'ont été testés que sur des fibroblastes non-infectés, iii) les immunosuppresseurs, capables de moduler à la fois la réplication du VHC et l'activation des fibroblastes.

En outre, les fibroblastes intrahépatiques étant infectés *in vivo* et étant responsables de la fibrose hépatique, il est au moins aussi important de considérer les FIH que les hépatocytes pour tester la toxicité des médicaments qui vont être utilisés chez des patients porteurs du VHC. Un autre objet de l'invention concerne donc une méthode d'évaluation de la toxicité d'une molécule vis-à-vis de fibroblastes hépatiques de patient atteint de l'hépatite C comprenant les étapes suivantes :
- mise en présence de fibroblastes intrahépatiques infectés par un virus de l'hépatite C ou de fibroblastes intrahépatiques comprenant le génome d'un virus de l'hépatite C ou un réplicon de celui-ci, avec la molécule à tester ;
- évaluation de la mortalité desdits fibroblastes intrahépatiques mis en contact avec la molécule à tester.

On entend par mortalité cellulaire l'apoptose et/ou la nécrose des cellules. Les techniques permettant d'évaluer la mortalité cellulaire sont bien connues en elles-mêmes. Il s'agit notamment de la cytométrie en flux après marquage par l'annexine V et l'iodure de propidium, et de la technique TUNEL (immunocytochimie), techniques décrites dans l'article de Aoudjehane et al. (FASEB J., 21(7) : 1433-1444, 2007).

Avantageusement, les fibroblastes intrahépatiques sur lesquels la toxicité d'une molécule est testée sont des fibroblastes intrahépatiques infectés par le virus de l'hépatite C tels que définis précédemment.

La présente invention sera mieux comprise à l'aide du complément de description qui va suivre, qui se réfère à des exemples non-limitatifs illustrant notamment l'isolement de fibroblastes intrahépatiques (FIH) infectés *in vivo* par le virus de l'hépatite C, l'infection *in vitro* de FIH par le VHC, et la mise en évidence que l'infection des fibroblastes intrahépatiques par le VHC induit l'expression des collagènes I, III et IV.

### EXEMPLE 1 : ISOLEMENT ET CULTURE DE FIBROBLASTES INTRAHÉPATIQUES HUMAINS (FIH)

Des fibroblastes intrahépatiques (FIH) ont été isolés à partir de tissus hépatiques provenant soit de patients ayant subi une hépatectomie pour métastases ou tumeurs bénignes, soit de patients souffrant d'une infection chronique par le virus de l'hépatite C et ayant développé un hépatocarcinome ou un autre type de tumeurs. Les tissus hépatiques prélevés au cours d'une hépatectomie majeure l'ont été aussi loin que possible de la tumeur, en évitant la zone péritumorale immédiate. La dissociation des tissus a été réalisée par une méthode de perfusion de collagénase en deux étapes, les cellules étant séparées par centrifugation sur gradient, comme décrit par Hillaire et al. (Gastroenterology, 107 : 781-788, 1994).

Les vaisseaux visibles ont d'abord été perfusés à l'aide d'un tampon HEPES-EDTA, puis par la solution Liver Digest (Gibco, Cergy-Pontoise, France) comprenant 0,05% de collagénase, à raison de 10 ml par cathéter par minute durant 30 minutes. Les fragments de foie ont alors été agités doucement afin de libérer les cellules hépatiques détachées puis filtrés et centrifugés. Les hépatocytes ont été isolés à partir du culot, alors que les fibroblastes intrahépatiques ont été isolés à partir du surnageant. Les fibroblastes intrahépatiques ont été séparés des autres cellules du surnageant par centrifugation à 1800rpm pendant 10 minutes comme décrit par Win Km et al. (Hepatology, 18 : 137-145, 1993). La viabilité cellulaire a été déterminée par le test de l'exclusion au bleu trypan, et les fibroblastes intrahépatiques ont été ensemencés en boîte de culture en milieu DMEM (Gibco) supplémenté avec 10% de sérum de veau foetal, 100U/ml de pénicilline et 100mg/ml de streptomycine et cultivés à 37°C sous atmosphère 5% CO2. Le milieu de culture a été remplacé un jour après l'ensemencement. A confluence, les cellules sont cultivées à nouveau et maintenues dans une flasque de 75cm².

La pureté des fibroblastes intrahépatiques en culture a été analysée par cytométrie en flux, immunohistochimie et immunofluorescence comme décrit précédemment par Aoudjehane et al. (Lab. Invest., 88 : 973-985, 2008). Ainsi, la pureté et l'activation des fibroblastes intrahépatiques ont été évaluées en mesurant l'expression de l'alpha-SMA, marqueur des cellules hépatiques étoilées, et de la vimentine, un marqueur des cellules d'origine mésenchymateuse. En outre, les fibroblastes intrahépatiques ont également été caractérisés par cytométrie en flux et immunofluorescence en mesurant l'expression de CD90, un marqueur des fibroblastes, et de CD31, un marqueur des cellules endothéliales. Plus de 95% des cellules possédaient le marqueur CD90 et seulement 1% des cellules présentaient le marqueur CD31, ce qui indique que la culture de fibroblastes intrahépatiques isolés par la méthode décrite ci-dessus est presque pure.

Dans les expériences décrites ci-après, seules les cellules issues des passages deux à six ont été utilisées. De plus, chaque expérience a été réalisée à partir de cellules provenant d'au moins six foies humains différents.

### EXEMPLE 2 : EVALUATION DE L'EXPRESSION DE CD81 ET DU RECEPTEUR AU LDL (LDL-R) À LA SURFACE DES FIBROBLASTES INTRAHÉPATIQUES HUMAINS (FIH)

L'expression de CD81 et du récepteur LDL-R a été évaluée par immunohistochimie et cytométrie en flux.

### 2.1. Evaluation par immunohistochimie

Les fibroblastes intrahépatiques ont été cultivés sur lamelle dans des plaques six puits (2.10⁴ cellules par puits) dans du milieu DMEM (Gibco) supplémenté avec 10% de sérum de veau foetal puis fixés dans du PBS contenant 4% de paraformaldéhyde. Alternativement, lorsque les cellules sont cultivées sur plaque, les cellules sont remises en suspension dans du PBS puis cytocentrifugées sur des lamelles SuperFrost Plus Slides (CML, Nemours, France). Les cellules sont alors séchées à l'air durant la nuit à température ambiante, fixées durant 10 minutes à l'acétone, puis utilisées directement ou stockées à -20°C jusqu'à l'analyse par immunohistochimie.

L'expression de la molécule de surface CD81 et du récepteur LDL-R a été étudiée par une technique immunoenzymatique indirecte utilisant un complexe phosphatase alcaline / anti-phosphatase alcaline comme décrit précédemment par Conti et al. (Transplantation, 76 : 210-216, 2003). Brièvement, la détection de CD81 a été réalisée à l'aide d'un anticorps monoclonal anti-CD81 dilués au 1/50 utilisé comme anticorps primaire et d'un IgG de lapin anti-souris utilisé comme anticorps secondaire. Pour détecter le récepteur LDL-R, des anticorps polyclonaux anti-LDL-R dilués au 1/10 et des IgG de souris anti-lapin ont été utilisés respectivement comme anticorps primaire et secondaire. La lamelle a ensuite été incubée en présence de complexes phosphatase alcaline/anti-phosphatase alcaline, et l'activité phosphatase alcaline a été révélée 20 minutes dans des solutions de fast-red TR (1 mg/ml) et de naphthol phosphate (0,2mg/ml) (Sigma, Saint Quentin-Fallavier, France) contenant 0,24 g/ml de lévamisole. Pour finir, les lamelles ont été contrecolorées à l'hématoxyline (coloration nucléaire). Des témoins négatifs, pour lesquels l'anticorps primaire avait été omis ou remplacé par un anticorps non-spécifique, ont également été analysés. En outre, un témoin positif, à savoir des cellules PBMC qui exprime naturellement à leur surface la molécule CD81 et le LDL-R, a également été analysé.

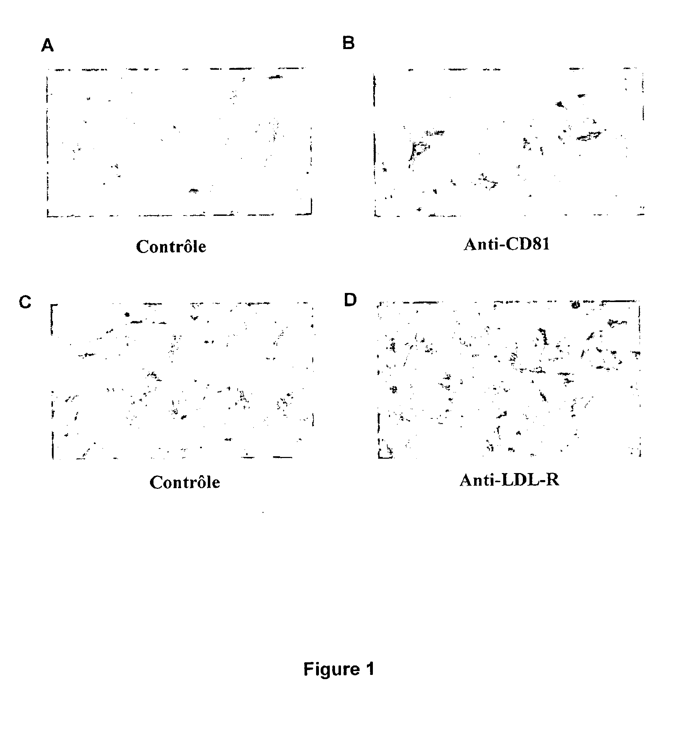

Les résultats de l'étude par immunohistochimie sont présentés sur la Figure 1. Les Figures 1.A à 1.C sont des photographies en noir et blanc de fibroblastes intrahépatiques fixés sur lames témoins (non traitées par les anticorps anti-CD81 ou anti-LDL-R), les Figures 1.B et 1.D représentent les résultats obtenus pour les fibroblastes intrahépatiques fixés sur lames traitées respectivement par les anticorps anti-CD81 et anti-LDL-R.

Cette Figure montre que les cellules témoins ne sont pas colorées (elles apparaissent gris clair sur les Figures 1.A et 1.C) alors que les cellules mises en présence d'un anticorps anti-CD81 ou d'un anticorps anti-LDL-R sont colorées (elles apparaissent gris foncé sur les Figures 1.B et 1.D), ce qui indique que les anticorps se sont fixés à la surface cellulaire.

Ces résultats indiquent donc que les fibroblastes intrahépatiques expriment à la surface cellulaire la molécule CD81 et le LDL-R, les deux récepteurs putatifs du VHC.

Il est à noter que comme attendu les cellules PBMC (témoin positif) ont été marquées par les anticorps anti-CD81 et anti-LDL-R (résultats non présentés).

### 2.2. Evaluation par cytométrie en flux

Les niveaux d'expression de la molécule CD81 et du LDL-R ont également été analysés par cytométrie en flux. Pour ce faire, les cellules ont été mises en présence d'un anticorps de lapin anti-LDL-R humain ou d'un anticorps de souris anti-CD81 humain durant 60 minutes à 4°C. Les cellules ont ensuite été lavées et incubées respectivement avec un anticorps secondaire anti-lapin ou anti-souris. Les cellules ont ensuite été lavées dans du PBS puis fixées dans du PBS contenant 4% de paraformaldéhyde. Un anticorps conjugué à un fluorochrome non-spécifique de CD81 ou LDL-R a été utilisé comme témoin négatif. Les cellules ont ensuite été analysées par FACS. Les expériences ont été réalisées au moins 3 fois.

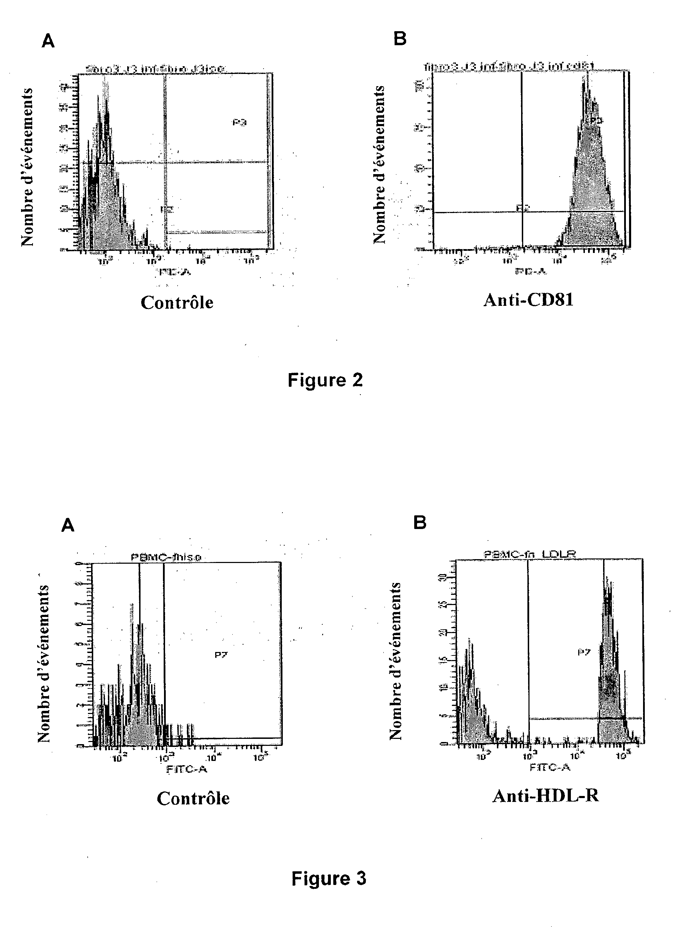

Les résultats de l'analyse par cytométrie de flux obtenus pour CD81 et LDL-R sont présentés respectivement sur les Figures 2 et 3. Sur ces Figures, le nombre d'événements (nombre de cellules) est indiqué en ordonnée, l'intensité de fluorescence (correspondant au marquage des cellules par l'anticorps anti-CD81 ou l'anticorps anti-LDL-R) est représentée en abscisse.

L'analyse du pic de distribution indique que plus de 95% des fibroblastes intrahépatiques sont marqués par l'anticorps anti-CD81 (cf. Figure 2.A),et plus de 70% sont marqués par l'anticorps anti-LDL-R (cf. Figure 3.A), alors que les témoins négatifs ne présentent aucun marquage (Figures 2.B et 3.B).

Ces résultats confirment que les fibroblastes intrahépatiques expriment à leur surface cellulaire le CD81 et le LDL-R.

### EXEMPLE 3 : EVALUATION DE L'INFECTION DES FIBROBLASTES INTRAHÉPATIQUES HUMAINS (FIH) PAR LE VHC

### 3.1. Préparation de stocks de VHC-JFH1

Des lignées hépatocellulaires Huh-7 ou Huh-7.5.1 (Zhong et al. P.N.A.S., 102 : 9294-9299), ont été cultivées à 37°C sous atmosphère humide et 5% de CO₂ dans du milieu DMEM (Invitrogen) supplémenté avec 10mM de HEPES (pH 7,3), des acides aminés non essentiels (Invitrogen), 2 mM de L-glutamine (Invitrogen), et 10% de sérum de veau foetal inactivé. Les particules virales VHC issues de l'infection de cellules Huh-7 ont été préparées en utilisant la méthode décrite par Wakita et al. (Nat. Med., 11 : 791-796, 2005). Brièvement, les cellules ont été transf-ectées avec l'ARN génomique obtenu par transcription *in vitro* à partir du plasmide pJFH1. Le surnageant de culture est prélevé après plusieurs passages lorsque le titre viral atteint au moins 1.10⁵ unités formant des foyers par ml. Le titrage est réalisé comme décrit par Pène et al. (J. Virol. Hepat., 2009). Pour certaines préparations de VHC- JFH1, une amplification additionnelle du virus a été réalisée en infectant des cellules Huh-7.5.1. Les stocks de surnageant contenant les virus ont été filtrés sur membranes 0,45µm puis concentrés par ultrafiltration sur membrane ayant un seuil de coupure de 100 000 daltons. Les stocks viraux ont alors été aliquotés et stockés à - 80°C. Les stocks ont été titrés comme décrit par Pène et al. (J. Virol. Hepat., 2009). Brièvement, des cellules Huh-7.5.1 ont été cultivées en présence de dilution en série des stocks viraux filtrés, puis, 3 jours post-inoculation, l'infection des cellules a été évaluée par immunofluorescence *in situ.* Les cellules infectées par le VHC apparaissent comme de petits amas cellulaire, chaque amas étant considéré comme un foyer infectieux. Le titre infectieux est exprimé en unités formant des foyers (ci-après abrégé en « ffu ») par ml de stocks, déterminé par le nombre de foyers à la dilution la plus importante.

### 3.2. Infection de fibroblastes intrahépatiques par le VHC

Les fibroblastes intrahépatiques ont été infectés un jour après leur mise en culture par le VHC-JFH1 à une multiplicité d'infection de 0,1 ffu par cellule. Après une nuit d'incubation à 37°C, les monocouches cellulaires ont été lavées trois fois à l'aide d'un tampon phosphate salin puis cultivées en conditions standard (Aoudjehane et al., Lab. Invest., 88 : 973-985, 2008) dans du milieu DMEM (Gibco) 10% SVF en présence de pénicilline et de streptomycine (1%) ainsi que de pyruvate (1%).

### A. Analyse de la réplication du génome du VHC dans les fibroblastes intrahépatiques

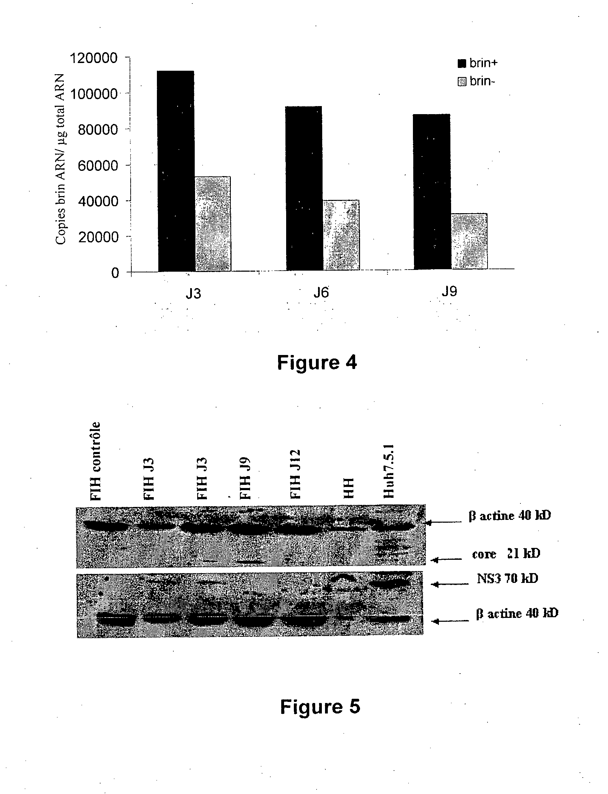

Afin de déterminer si le génome du VHC est capable de se répliquer dans des fibroblastes intrahépatiques inoculés par des VHC-JFH1, les brins d'ARN positif (ARN génomique) et les brins d'ARN négatif (ARN anti-sens) ont été quantifiés. Pour cela, les cellules ont été prélevées à J3, J6 et J9 post-infection, lavées dans du tampon PBS, puis préparées à l'aide du kit d'extraction « RNeasy minikit » (QIAGEN S.A, Courtaboeuf, France) selon les recommandations du fournisseur, les cellules ayant été lysées dans 300 µl de tampon pour 3.10⁵ cellules. Les ARN intracellulaires ont été quantifiés par une RT-PCR quantitative spécifique de chaque brin comme décrit par Carriere et al. (J.Med. Virol., 79 : 155-160, 2007). Les résultats de cette quantification sont présentés sur la Figure 4.

La Figure 4 est un histogramme montrant les résultats obtenus pour la quantification du brin d'ARN positif (barres noires) et la quantification du brin d'ARN négatif (barres grises). Le nombre de copies du brin d'ARN par µg d'ARN total est indiqué en ordonnée, le nombre de jours post-inoculation est indiqué en abscisse.

Ces résultats montrent que les ARN positifs, mais également les ARN négatifs (ARN présents uniquement lorsque la réplication du VHC a lieu), sont détectables dès 3 jours post-inoculation. Ceci indique clairement que le VHC est capable de se répliquer efficacement dans des fibroblastes intrahépatiques.

### B. Analyse de l'expression du génome du VHC dans les fibroblastes intrahépatiques

Pour déterminer si la polyprotéine du VHC est traduite et maturée dans les fibroblastes intrahépatiques, l'expression de la protéase NS3 et de la protéine de capside du VHC a été analysée par transfert de Western. Des fibroblastes intrahépatiques infectés ou non-infectés (contrôle) ont été lavés dans du PBS froid puis lysés dans les boîtes de culture par ajout de tampon Laemmli comprenant 1,2% de β-2 mercaptoéthanol (40mM de tris-HCl, pH 6,8, 5mM de DTT, 1% de SDS, 7,5% de glycérol et 0,01% de bleu de bromophénol). Après avoir fait bouillir le lysat cellulaire, les échantillons (comprenant 50 mg de protéines totales par puits) ont été mis à migrer sous conditions réductrices sur gel de polyacrylamide 15%-dodécyl-sulfate de sodium (pour l'analyse de la protéine de caspide) ou sur gel de polyacrylamide 10% (pour l'analyse de la protéine NS3), puis transférés sur membrane de nitrocellulose. Les membranes de nitrocellulose ont été saturées dans du PBS comprenant 5% de lait, 0,1% de Tween 20 pendant 1 heure, puis incubées durant 1 heure avec l'anticorps monoclonal de souris anti-capside de VHC (anticorps C7-50, Alexis Biochemicals) dilué au 1 : 1000 ou avec un anticorps de souris anti-NS3 de VHC (anticorps 1847, Virostat, Portland, MA) dilué au 1 : 100. Les membranes ont alors été lavées, incubées pendant 1 heure avec un anticorps secondaire anti-souris couplé à la peroxydase dilué au 1 : 5000, lavées puis incubées avec un réactif chimioluminescent (Pierce, Rockford, IL, USA). Les résultats sont représentés sur la Figure 5.

La Figure 5 montre que la protéine de capside (« core ») est détectable à J3 et J9. Cette protéine apparaissant sous forme d'une seule bande de 21 kDa, il s'agit vraisemblablement de la forme mature de la capside. La protéase NS3 est détectable à J3 et possède la masse moléculaire attendue (70kDa).

Cette expérience confirme que la polyprotéine du VHC est exprimée et correctement maturée dans des fibroblastes intrahépatiques inoculés par le VHC-JFH1.

### C. Analyse du potentiel infectieux des surnageants de culture

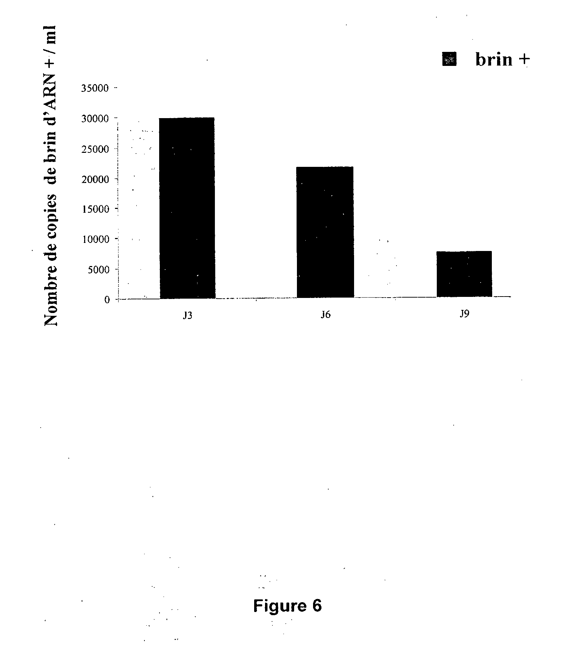

Les résultats précédents démontrant que le génome du VHC est capable de se répliquer et d'exprimer les protéines du VHC dans les fibroblastes intrahépatiques, la capacité du VHC à se multiplier et à produire de nouvelles particules infectieuses a été étudiée. Pour ce faire, la présence d'ARN positif et négatif dans le surnageant de culture de cellules infectées comme décrit précédemment a été déterminée. La Figure 6 est un histogramme montrant les résultats obtenus pour la quantification du brin d'ARN positif. Le nombre de copies du brin d'ARN positif par ml de surnageant est indiqué en ordonnée, le nombre de jours post-inoculation est indiqué en abscisse. A trois jours post-infection, l'ARN positif (ARN génomique) a été mis en évidence, la quantité décroissant avec le temps. Au contraire, le brin d'ARN négatif, qui n'est jamais présent dans les particules virales, n'a pas été détecté. Ceci indique que l'ARN génomique présent dans le surnageant n'a pas été relargué dans le milieu par un mécanisme non-spécifique, sans quoi l'ARN négatif aurait vraisemblablement aussi été détecté.

Le surnageant s'est cependant révélé non-infectieux lors de l'inoculation de cellules Huh-7.5.1.

### EXEMPLE 4 : NEUTRALISATION DE L'INFECTION DES FIBROBLASTES INTRAHÉPATIQUES HUMAINS (FIH)

### 4.1. Test de neutralisation par des anticorps anti-CD81

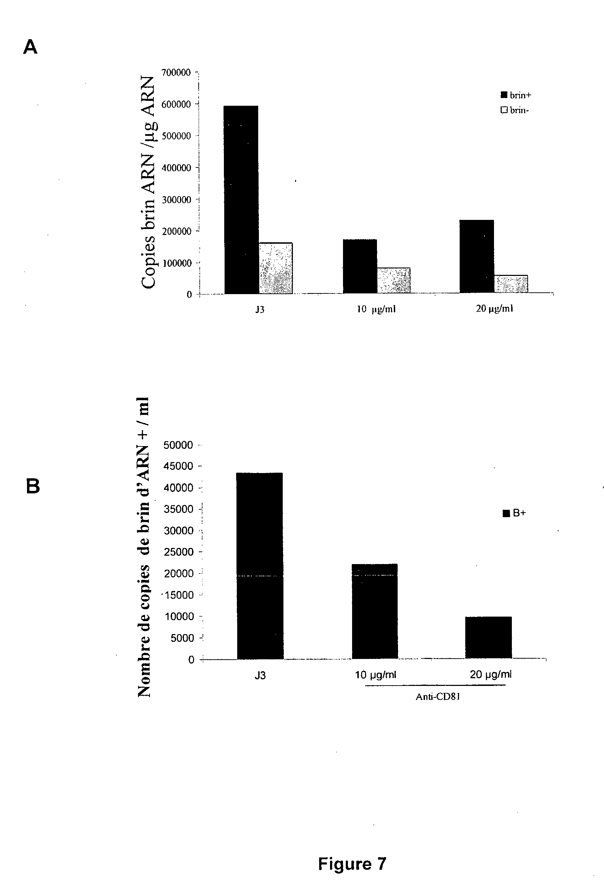

Nombre de résultats publiés ces dernières années montrent que la molécule CD81 serait un récepteur de surface du VHC essentiel à sa fixation à la cellule et à son entrée. Afin de déterminer si l'infection des fibroblastes intrahépatiques par le VHC-JFH1 dépend de la molécule de surface CD81, des fibroblastes intrahépatiques ont été pré-incubés durant une heure à 37°C avec des quantités croissantes d'anticorps monoclonaux de souris anti-CD81 humain ou d'anticorps non-spécifiques (contrôle). Les cellules ont été lavées puis infectées par le VHC-JFH1 à une multiplicité d'infection de 0,1 ffu par cellule. Afin d'évaluer le pourcentage de neutralisation, les quantités d'ARN positif et négatif intracellulaires, ainsi que la quantité d'ARN positif extracellulaire dans le surnageant, ont été déterminées à trois jours post-infection comme décrit au point 3.2.A. Les résultats sont présentés sur la Figure 7.

La figure 7.A est un histogramme montrant les résultats obtenus pour la quantification du brin d'ARN positif (barres noires) et la quantification du brin d'ARN négatif (barres grises) intracellulaires. Le nombre de copies du brin d'ARN par µg d'ARN total est indiqué en ordonnée, la concentration en anti-CD81 est indiquée en abscisse. Cette Figure montre une forte réduction de la quantité d'ARN brin positif et d'ARN brin négatif lorsque les cellules sont pré-incubées avec 10 µg d'anti-CD81 par ml de milieu de culture par rapport au contrôle : fibroblastes intrahépatiques infectés incubés avec un anticorps non-spécifique prélevés à 3 jours post-inoculation (barres J3). De même, la Figure 7.B qui est un histogramme représentant les résultats obtenus pour la quantification du brin d'ARN positif extracellulaire montre que la quantité d'ARN positif dans le surnageant de culture décroît de façon dose dépendante.

Ces résultats montrent que l'infection des fibroblastes intrahépatiques par le VHC-JFH1 est majoritairement dépendante de la présence de CD81 à la surface des cellules, ce qui indique que le VHC infecte les cellules en utilisant le CD81 comme principal récepteur cellulaire.

### 4.2. Test de neutralisation par l'interféron-alpha

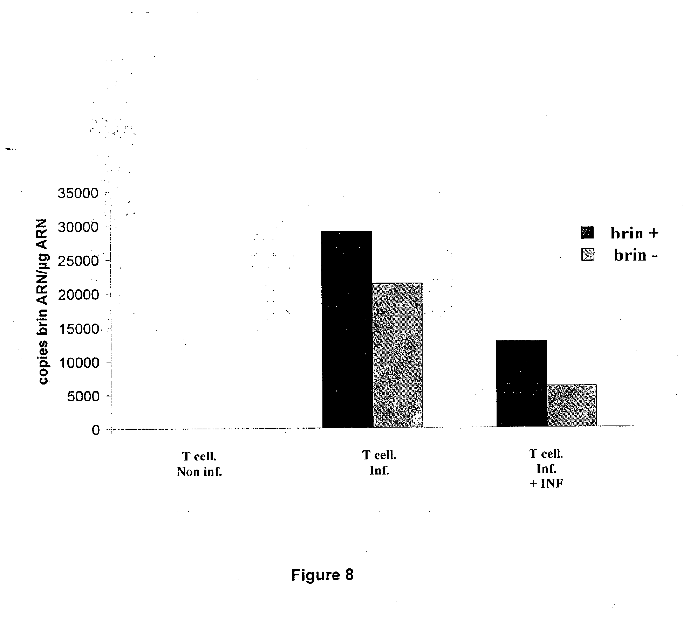

L'interféron-alpha inhibant la réplication du VHC dans des hépatocytes infectés *in vitro* et étant utilisé depuis de nombreuses années pour traiter les patients atteints d'hépatite C, des fibroblastes intrahépatiques ont été pré-incubés durant une heure à 37°C en présence de 500 U/ml d'interféron-alpha, lavés puis infectés par le VHC-JFH1 à une multiplicité d'infection de 0,1 ffu par cellule. Les résultats sont présentés sur la Figure 8. Cette Figure montre que les quantités d'ARN brin positif et d'ARN brin négatif diminuent lorsque les cellules sont pré-incubées avec l'interféron-alpha.

### EXEMPLE 5 : EVALUATION DE L'APOPTOSE INDUITE LORS DE L'INFECTION DES FIBROBLASTES INTRAHÉPATIQUES HUMAINS (FIH) PAR LE VHC

Afin de déterminer si l'infection par le VHC induit l'apoptose des fibroblastes intrahépatiques, un test *in vitro* d'apoptose Annexine V-FITC a été réalisé en utilisant le kit Annexine V-FITC (Immunotech, Marseille, France) selon les instructions du fournisseur. Ce test repose sur l'externalisation de la phosphatidylsérine par les cellules apoptotiques, et sur la fixation de l'Annexine V-FITC sur cette molécule. La nécrose a également été déterminée en effectuant une contre-coloration à l'iodure de propidium. Après 48 heures de culture, les fibroblastes intrahépatiques ont été soit infectés par le VHC-JFH1 à une multiplicité d'infections de 0,1 ffu par cellule, soit traités avec 20 µM de C2-Ceramide(Sigma)(témoin positif d'apoptose). Après 3, 6 et 9 jours post-infection, les cellules ont été mises en suspension dans un tampon de liaison contenant du Ca²⁺ et incubées avec 1 µg/ml d'Annexine C-FITC et 1 µg/ml d'iodure de propidium. Le signal émis par la FITC (fluorescéine isothiocyanate) a alors été mesuré par cytométrie en flux afin de déterminer l'apoptose, alors que la nécrose a été déterminée par mesure du nombre de cellules marquées à l'iodure de propidium. Il s'est avéré que l'infection par le VHC-JFH1 induit l'apoptose d'un nombre minime de fibroblastes intrahépatiques et que l'infection n'est pas toxique pour les fibroblastes intrahépatiques (résultats non présentés).

### EXEMPLE 6 : EFFET DE L'INFECTION PAR LE VHC SUR LA PROLIFÉRATION DES FIBROBLASTES INTRAHÉPATIQUES HUMAINS ET L'EXPRESSION DES GENES IMPLIQUÉS DANS LA FIBROSE

### 6.1. Evaluation de la prolifération des fibroblastes intrahépatiques suite à l'infection par le VHC

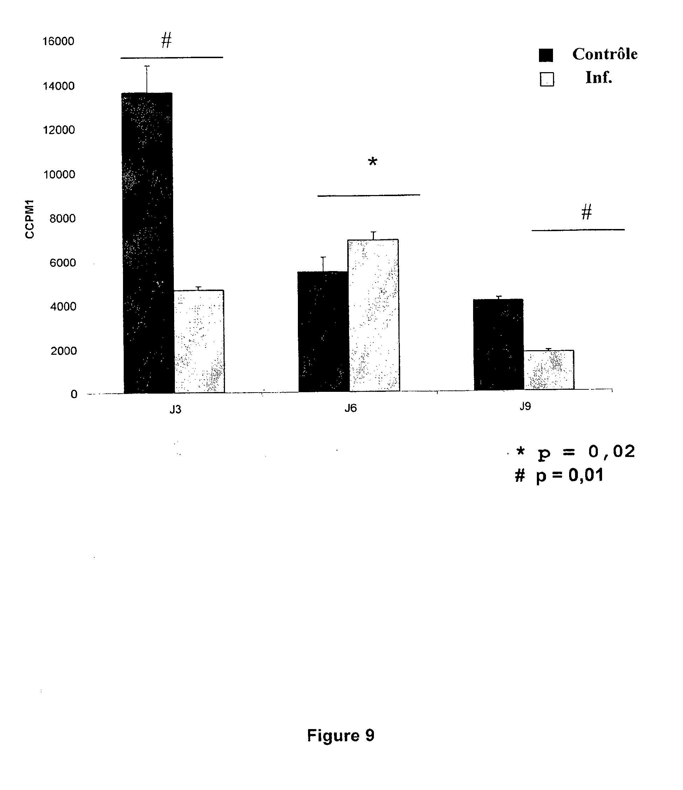

Les fibroblastes intrahépatiques ont été ensemencés en plaques 96 puits (5.10⁴ cellules par puits) dans du milieu DMEM (Gibco) sans sérum. A 48 post-ensemencement, les cellules ont été infectées par le VHC-JFH1 à une multiplicité d'infections de 0,1 ffu par cellule. Un témoin cellules non-infectées a également été réalisé. La prolifération cellulaire a été mesurée après 16 heures de culture en présence de thymidine tritié [3H] (1 µCi/puits) à l'aide d'un compteur à scintillation. Ces résultats sont présentés sur la Figure 9.

La figure 9 est un histogramme dans lequel le témoin cellules non-infectées est représenté par les barres noires et les essais concernant les cellules infectées sont représentés par les barres grises. Le nombre de coups par minute (« CCPM1 ») est indiqué en ordonnée, le nombre de jours post-infection est précisé en abscisse. Cette Figure montre que la prolifération est significativement inhibée (P=0,01) à trois jours post-infection par rapport au témoin cellules non-infectées. Cependant, à 6 jours post-infection les cellules infectées prolifèrent significativement plus que le témoin (p=0,02). A 9 jours post-infection, les cellules sont confluentes et les résultats ne sont plus interprétables.

### 6.2. Analyse de l'expression des gènes du collagène de type I et de type IV, et de l'alpha-SMA dans les fibroblastes intrahépatiques infectés par le VHC

L'expression des gènes de la GAPDH, du collagène de type I, du collagène de type IV, de l'alpha-SMA, ainsi que l'expression des gènes de l'HNF-1β, du Cytochrome P450 et de l'albumine ont été analysées par RT-PCR en utilisant le kit « DNA Fast Start SYBR green » (Roche Diagnosics), et l'appareil LightCycler® (Roche Diagnosics), dans les fibroblastes intrahépatiques non-infectés (contrôle) ou infectés par le VHC.

L'amplification PCR a été réalisée dans un volume total de 20 µl/ml en capillaire contenant 20 ng de chaque amorce des paires d'amorces décrites dans le Tableau I ci-dessous (Sigma-Genosys Ltd), 3 mM de MgCl₂, 2 µl de composition Light Cycler Fast Start Master SYBR Green (contenant 1,25 unité de polymérase Fast StartTaq, du tampon Taq 10X, 2 mM de chacun des dNTP, 10 µl de SYBR Breen 10X (Roche Diagnotics) et 2 µl d'ADNc (préalablement dilué au 1/10) issu de chaque souche et obtenu comme décrit par Zhong et al. (P.N.A.S., 102 : 9294-9, 2005) et *al.* (J. Med. Virol., 79 : 155-60, 2007).

**Tableau I**

| **Gène** | **Amorces :** | **séquence sens (+)** | **SEQ ID (#)** |
|---|---|---|---|
| | | **Séquence anti-sens (-)** | |
| GAPDH | (+) 5'-ACAGTCCATGCCATCACTGCC-3' | | **(1)** |
| | (-) 5'-GCCTGCTTCACCACCTTCTTG-3' | | **(2)** |
| collagène de type I | (+) 5'-CCTCAAGGGCTCCAACGAG-3' | | **(3)** |
| | (-) 5'-TCAATCACTGTCTTGCCCCA-3' | | **(4)** |
| collagène de type IV | (+) 5'-ATGTCAATGGCACCCATCAC-3' | | **(5)** |
| | (-) 5'-CTTCAAGGTGGACGGCGTAG-3' | | **(6)** |
| alpha-SMA | (+) 5'-TGAAGAGCATCCCACCCT-3' | | **(7)** |
| | (-) 5'-ACGAAGGAATAGCCACGC-3' | | **(8)** |
| HNF-1β | (+) 5'-GAAACAATGAGATCACTTCCTC-3' | | **(9)** |
| | (-) 5'-CTTTGTGCAATTGCCATGACTC-3' | | **(10)** |
| Cytochrome P450 | (+) 5'-AGCACAACTCTGAGATATGG-3' | | **(11)** |
| | (-) 5'-ATAGTCACTGTACTTGAACT-3' | | **(12)** |
| Albumine | (+) 5'-TTAGGATCCCCCAGGAAGACATCCTTTGC-3' | | **(13)** |
| | (-) 5'-CCTGAGCCAGAGATTTCC-3' | | **(14)** |

L'amplification a été conduite dans les conditions suivantes :
- dénaturation initiale à 94°C pendant 10 min,
- 40 cycles d'amplification comprenant chacun 3 phases :
   i) 95°C pendant 10 sec (dénaturation) ;
   ii) hybridation des amorces avec l'ADN pendant 5 sec à 70°C pour la GAPDH, 58°C pour le collagène de type I, 65°C pour le collagène de type IV, 63°C pour l'alpha-SMA, 66°C pour l'HNF-1β, 60°C pour le CP450, 70°C pour l'albumine ;
   iii) 72°C pendant 5 sec (polymérisation).

Le niveau d'expression relatif des ARNm du collagène IV, de l'alpha-SMA, de l'HNF-1β, du CP450 et de l'albumine a été calculé en utilisant la méthode du « 2^{ΔΔCT}» décrite par Livak et Schmittgen (Methods, 25 : 402-408, 2001).

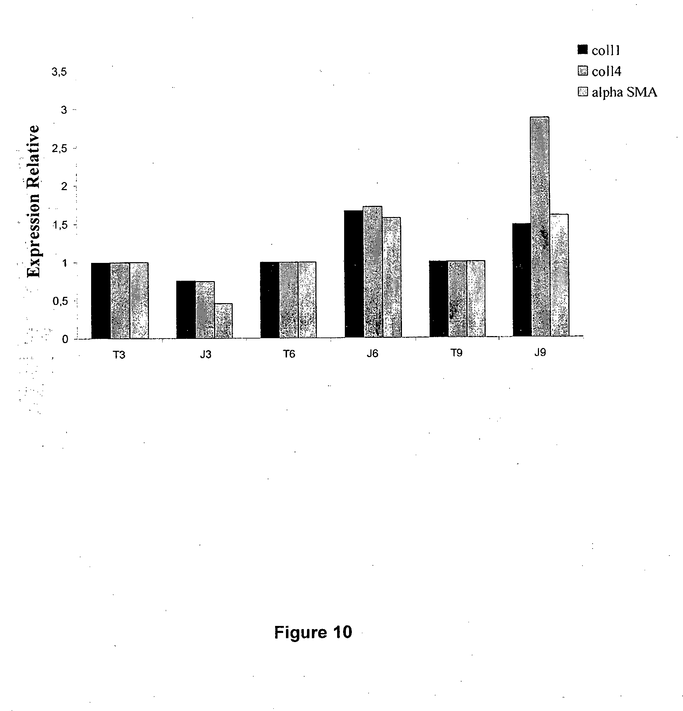

Les résultats de cette expérience sont présentés sur la Figure 10. L'expression du collagène de type I, du collagène de type IV et de l'alpha-SMA est représentée respectivement par les barres noires, gris foncé et gris clair. L'expression relative est indiquée en ordonnée. L'origine de l'échantillon, à savoir cellules non-infectées (Témoin) ou cellules infectées, est précisé en abscisse (« T » pour témoin et « J » pour les essais cellules infectées). Le jour post-infection où l'échantillon a été prélevé est également précisé. Cette Figure montre qu'à J3, l'expression du collagène de type I, du collagène de type IV et de l'alpha-SMA par les cellules infectées est inférieure à celle des cellules non-infectées. Cependant, à 6 et 9 jours post-infection, l'expression de ces trois gènes est significativement supérieure à celle du témoin cellules non-infectées (p=0,02).

L'ensemble des résultats présentés dans l'exemple 6 indique que chez les fibroblastes intrahépatiques infectés par le VHC-JFH1 la prolifération et l'activation cellulaire sont dans un premier temps inhibées, pour ensuite être augmentées.

### EXEMPLE 7 : ISOLEMENT DE FIBROBLASTES INTRAHÉPATIQUES HUMAINS INFECTÉS IN VIVO PAR LE VHC

Des fibroblastes intrahépatiques ont été isolés à partir de foie de patients infectés par le VHC comme décrit dans l'exemple I. La pureté des fibroblastes intrahépatiques a été évaluée en mesurant l'expression de l'alpha-SMA, marqueur des cellules hépatiques étoilées, et de la vimentine, un marqueur des cellules d'origine mésenchymateuse. En outre, les fibroblastes intrahépatiques ont également été caractérisés en mesurant l'expression de CD90 et de CD31. Afin d'exclure une contamination des fibroblastes intrahépatiques par des hépatocytes infectés, les ARNm de trois gènes spécifiquement exprimés par les hépatocytes ont été quantifiés. Ces trois gènes sont :
i) le gène du facteur de transcription HNF-4 (« hepatocyte nuclear factor »), régulateur clef dans le maintien du phénotype hépatocyte ;
ii) le gène de l'albumine, qui est exprimé dans les hépatocytes primaires ;
iii) le gène CYP2E1, un marqueur de la fonction de détoxification des hépatocytes.

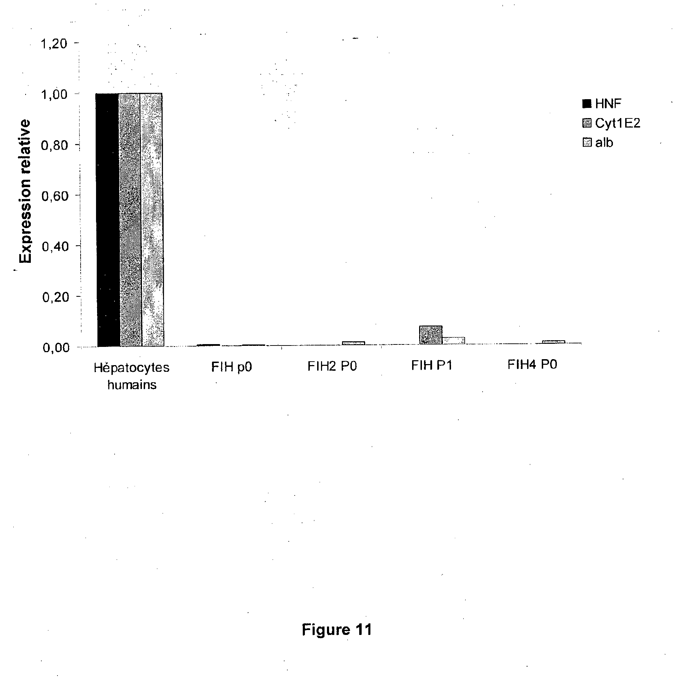

Les résultats sont présentés sur la Figure 11. Le nombre de passages est précisé en abscisse (« p0 », « p1 »). FIH, FIH2 et FIH4 représentent différents lots de fibroblastes intrahépatiques. Comme le montre cette Figure, alors que le témoin hépatocyte humain (« HH ») exprime chacun de ces gènes, les fibroblastes intrahépatiques n'expriment aucun de ces 3 gènes, même après un passage.

Les fibroblastes intrahépatiques isolés à partir du foie de patients infectés par le VHC n'ont donc pas été contaminés par des hépatocytes infectés lors de leur isolement.

Afin de déterminer si les fibroblastes intrahépatiques isolés à partir de patients VHC positifs sont infectés par le VHC, les ARN positifs et négatifs intracellulaires du VHC ont été quantifiés comme décrit dans l'exemple 3.2.A. En outre, l'ARN positif a été quantifié dans le surnageant.

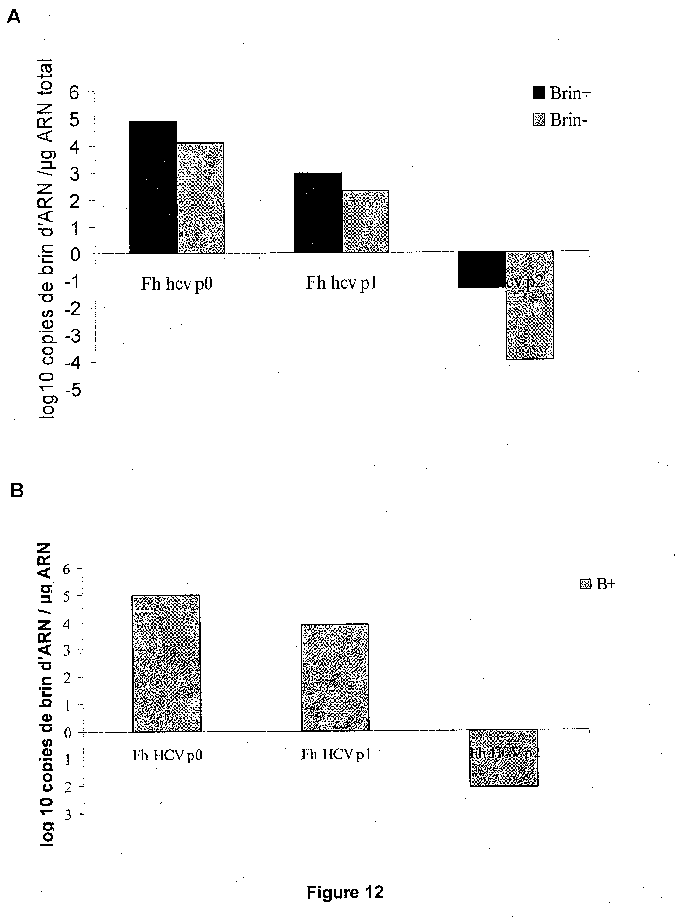

Les résultats sont présentés à la Figure 12. Dans la figure 12.A, qui représente les résultats de la quantification des ARN viraux intracellulaires, le nombre de copies d'ARN positif (barres noires) et d'ARN négatif (barres grises) est indiqué en ordonnée (en log10 par µg d'ARN total), le nombre de passages est précisé en abscisse (« p0 », « p1 », « p2 »). Cette Figure montre que les fibroblastes intrahépatiques isolés expriment environ 1.10⁵ brins d'ARN positif par µg d'ARN total et sont donc bien infectés *in vivo* par le VHC. En outre, la détection de 1.10⁴ brins d'ARN négatif par µg d'ARN total montre bien que le VHC se réplique dans les fibroblastes intrahépatiques *in vivo.* Après un passage des cellules, le nombre d'ARN positif décroît, et continue à décroître au cours des passages successifs.

La Figure 12.B montre les résultats obtenus pour la quantification du brin d'ARN positif dans le surnageant. Le nombre de copies du brin d'ARN positif par ml de surnageant est indiqué en ordonnée, le nombre de passages est indiqué en abscisse. L'ARN génomique est bien présent dans le surnageant, l'évolution du nombre de brins positifs suivant celle observée dans le cas des ARN viraux intracellulaires.

## Revendications

1. Méthode d'obtention de fibroblastes intrahépatiques infectés par le virus de l'hépatite C, comprenant une étape de mise en contact *in vitro* de fibroblastes intrahépatiques avec des particules infectieuses du virus de l'hépatite C.

2. Méthode selon la revendication 1, **caractérisée en ce que** le virus de l'hépatite C est le VHC-JFH1.

3. Méthode d'obtention de fibroblastes intrahépatiques infectés par le virus de l'hépatite C, comprenant une étape d'isolement des fibroblastes intrahépatiques à partir d'un échantillon d'un tissu hépatique préalablement prélevé sur un patient VHC-positif.

4. Fibroblaste intrahépatique isolé infecté par un virus de l'hépatite C, ou comprenant le génome d'un virus de l'hépatite C ou un réplicon de celui-ci, susceptible d'être obtenu par l'une quelconque des méthodes définies aux revendications 1 à 3.

5. Procédé de réplication *in vitro* du génome du virus de l'hépatite C, **caractérisé en ce qu'**il comprend une étape de culture *in vitro* de fibroblastes intrahépatiques isolés infectés par le virus de l'hépatite C.

6. Procédé selon la revendication 5, **caractérisé en ce que** les fibroblastes intrahépatiques isolés infectés par le virus de l'hépatite C sont obtenus par l'une quelconque des méthodes définies aux revendications 1 à 3.

7. Méthode de criblage de molécules anti-VHC comprenant les étapes suivantes :
- mise en présence de fibroblastes intrahépatiques infectés par un virus de l'hépatite C ou de fibroblastes intrahépatiques comprenant le génome d'un virus de l'hépatite C ou un réplicon de celui-ci, avec la molécule à tester ;
- mesure de la réplication du génome du VHC et/ou de la production de particules du VHC.

8. Méthode de criblage selon la revendication 7, **caractérisée en ce que** l'étape de mesure de la réplication du génome du VHC est réalisée par quantification du brin d'ARN de polarité positive et/ou du brin d'ARN de polarité négative du VHC.

9. Méthode de criblage de molécules anti-fibrogénèse comprenant les étapes suivantes :
- mise en présence de fibroblastes intrahépatiques infectés par un virus de l'hépatite C ou de fibroblastes intrahépatiques comprenant le génome d'un virus de l'hépatite C ou un réplicon de celui-ci, avec la molécule à tester ;
- mesure de l'expression d'au moins un gène impliqué dans la fibrose.

10. Méthode de criblage selon la revendication 9, **caractérisée en ce que** le ou les gènes impliqué(s) dans la fibrose dont on mesure l'expression est/sont choisi(s) parmi les gènes des collagènes de type I, III, IV et V.

11. Méthode d'évaluation de la toxicité d'une molécule vis-à-vis de fibroblastes hépatiques de patient atteint de l'hépatite C comprenant les étapes suivantes :
- mise en présence de fibroblastes intrahépatiques infectés par un virus de l'hépatite C ou de fibroblastes intrahépatiques comprenant le génome d'un virus de l'hépatite C ou un réplicon de celui-ci, avec la molécule à tester ;
- évaluation de la mortalité desdits fibroblastes intrahépatiques.

## Patentansprüche

1. Verfahren zum Erhalt von mit Hepatitis C-Virus infizierten intrahepatischen Fibroblasten, umfassend einen Schritt des Inkontaktbringens von intrahepatischen Fibroblasten *in-vitro* mit infektiösen Hepatitis C-Viruspartikeln.

2. Verfahren nach Anspruch 1, **dadurch gekennzeichnet, dass** das Hepatitis C-Virus VHC-JFH1 ist.

3. Verfahren zum Erhalt von mit Hepatitis C-Virus infizierten intrahepatischen Fibroblasten, umfassend einen Schritt des Isolierens von intrahepatischen Fibroblasten aus einer Probe eines einem VHC-positiven Patienten zuvor entnommenen hepatischen Gewebes.

4. Isolierter intrahepatischer Fibroblast, der mit einem Hepatitis C-Virus infiziert ist oder das Genom eines Hepatitis C-Virus oder ein Replikon davon umfasst, erhältlich nach einem der Verfahren gemäß den Ansprüchen 1 bis 3.

5. Verfahren zur *in-vitro*-Replikation des Genoms eines Hepatitis C-Virus, **dadurch gekennzeichnet, dass** es einen *in-vitro*-Schritt des Kultivierens von isolierten, mit Hepatitis C-Virus infizierten intrahepatischen Fibroblasten umfasst.

6. Verfahren nach Anspruch 5, **dadurch gekennzeichnet, dass** die isolierten, mit Hepatitis C-Virus infizierten intrahepatischen Fibroblasten durch eines der Verfahren gemäß den Ansprüchen 1 bis 3 erhalten werden.

7. Screening-Verfahren für Anti-VHC-Moleküle, umfassend die folgenden Schritte:
- Inkontaktbringen von intrahepatischen Fibroblasten, die mit einem Hepatitis C-Virus infiziert sind, oder von intrahepatischen Fibroblasten, die das Genom eines Hepatitis C-Virus oder ein Replikon davon umfassen, mit dem zu testenden Molekül;
- Messen der Replikation des VHC-Genoms und/oder der Produktion von VHC-Partikeln.

8. Screening-Verfahren nach Anspruch 7, **dadurch gekennzeichnet, dass** der Schritt des Messens der Replikation des VHC-Genoms durch Quantifizierung des RNA-Strangs von VHC mit positiver Polarität und/oder des RNA-Strangs von VHC mit negativer Polarität erfolgt.

9. Screening-Verfahren für Anti-Fibrinogenese-Moleküle, umfassend die folgenden Schritte:
- Inkontaktbringen von intrahepatischen Fibroblasten, die mit einem Hepatitis C-Virus infiziert sind, oder von intrahepatischen Fibroblasten, die das Genom eines Hepatitis C-Virus oder ein Replikon davon umfassen, mit dem zu testenden Molekül;
- Messen der Expression wenigstens eines an der Fibrose beteiligten Gens.

10. Screening-Verfahren nach Anspruch 9, **dadurch gekennzeichnet, dass** das oder die an der Fibrose beteiligte(n) Gen(e), dessen/deren Expression gemessen wird, ausgewählt ist/sind aus Genen der Kollagene vom Typ I, III, IV und V.

11. Verfahren zum Beurteilen der Toxizität eines Moleküls gegenüber hepatischen Fibroblasten eines Patienten mit Hepatitis C, umfassend die folgenden Schritte:
- Inkontaktbringen von intrahepatischen Fibroblasten, die mit einem Hepatitis C-Virus infiziert sind, oder von intrahepatischen Fibroblasten, die das Genom eines Hepatitis C-Virus oder ein Replikon davon umfassen, mit dem zu testenden Molekül;
- Beurteilung der Mortalität der intrahepatischen Fibroblasten.

## Claims

1. Method of obtaining intrahepatic fibroblasts infected with hepatitis C virus comprising a step of bringing intrahepatic fibroblasts into *in vitro* contact with infectious particles of hepatitis C virus.

2. Method according to claim 1, **characterised in that** the hepatitis C virus is HCV JFH1.

3. Method of obtaining intrahepatic fibroblasts infected with hepatitis C virus comprising a step of isolating intrahepatic fibroblasts from a sample of hepatic tissue previously taken from an HCV-positive patient.

4. Isolated intrahepatic fibroblast infected with hepatitis C virus or comprising the genome of a hepatitis C virus or a replicon thereof, able to be obtained by any one of the methods defined in claims 1 to 3.

5. Process for *in vitro* replication of the genome of hepatitis C virus, **characterised in that** it comprises a step of *in vitro* culturing of isolated intrahepatic fibroblasts infected with hepatitis C virus.

6. Process according to claim 5, **characterised in that** the isolated intrahepatic fibroblasts infected with hepatitis C virus are obtained by any one of the methods defined in claims 1 to 3.

7. Method of screening anti-HCV molecules comprising the following steps:
- bringing of intrahepatic fibroblasts infected with a hepatitis C virus or intrahepatic fibroblasts comprising the genome of a hepatitis C virus or a replicon thereof into the presence of the molecule to be tested,
- measuring the replication of the HCV genome and/or the production of HCV particles.

8. Method of screening according to claim 7, **characterised in that** the step of measuring the replication of the HCV genome is performed by quantifying the positive-sense RNA strand of the HCV and/or the negative-sense RNA strand thereof.

9. Method of screening anti-fibrogenesis molecules comprising the following steps:
- bringing of intrahepatic fibroblasts infected with a hepatitis C virus or intrahepatic fibroblasts comprising the genome of a hepatitis C virus or a replicon thereof into the presence of the molecule to be tested,
- measuring the expression of at least one gene involved in fibrosis.

10. Method of screening according to claim 9, **characterised in that** the gene or genes involved in fibrosis whose expression is measured is/are selected from the genes of type I, III, IV and V collagens.

11. Method of evaluating the toxicity of a molecule to patient hepatic fibroblasts affected by hepatitis C comprising the following steps:
- bringing of intrahepatic fibroblasts infected with a hepatitis C virus or intrahepatic fibroblasts comprising the genome of a hepatitis C virus or a replicon thereof into the presence of the molecule to be tested,
- evaluating the mortality of the said intrahepatic fibroblasts.
